Europäisches Patentamt

European Patent Office

Office européen des brevets

(19)

(11) Publication number: **0 035 329**
**B1**

# EUROPEAN PATENT SPECIFICATION

(12)

(45) Date of publication of patent specification: **06.04.83**

(21) Application number: **81300532.9**

(22) Date of filing: **10.02.81**

(51) Int. Cl.³: **C 07 C 7/20, C 07 C 15/44, C 07 C 5/333**

(54) **Prevention of polymer formation in dehydrogenation of p-ethyltoluene.**

(30) Priority: **03.03.80 US 126247**

(43) Date of publication of application:
**09.09.81 Bulletin 81/36**

(45) Publication of the grant of the patent:
**06.04.83 Bulletin 83/14**

(84) Designated Contracting States:
**BE DE FR GB IT NL**

(56) References cited:
**DE - A - 1 567 839**
**US - A - 2 526 962**
**US - A - 3 222 263**

**Chemical Abstracts vol. 92, no. 12, 1980 Columbus, Ohio, USA Y.A. SANGALOV et al. "Effect of some sulfur-containing compounds on isobutylene polymerization in the presence of ethylaluminium dichloride" page 4, column 1, abstract no. 94742t**

(73) Proprietor: **MOBIL OIL CORPORATION**
**150 East 42nd Street**
**New York New York 10017 (US)**

(72) Inventor: **Chu, Chin Chiun**
**6 Nathan Drive**
**North Brunswick New Jersey 08902 (US)**

(74) Representative: **Cooper, John Anthony et al,**
**Mobil Court 3 Clements Inn**
**London WC2A 2EB (GB)**

Courier Press, Leamington Spa, England.

Prevention of polymer formation in dehydrogenation of p-ethyltoluene.

This invention is concerned with the catalytic dehydrogenation of p-ethyltoluene to p-methylstyrene. It is more particularly concerned with the prevention of polymer formation by the effluent gases in the condenser or cooling train.

Methyl styrene may be produced from ethyltoluene by dehydrogenation over catalysts such as ferric oxide or copper chromite. One problem which is encountered in this process is that the hot effluent containing the methyl styrene tends to form a proliferous polymer (often known, from its appearance, as "popcorn" polymer) in the condenser and other parts of the cooling train. This polymer tends to clog the apparatus and is therefore undesirable.

In the past, the problem of preventing the formation of proliferous polymers during the distillation of organic monomers has been encountered. For example, Canadian Patent No. 594,343 proposes to overcome this problem by adding various nitro compounds to vinyltoluene to inhibit the formation of polymer during distillation. Other typical inhibitors which have been proposed include nitrosoanilines, nitro and halo nitro substituted cresols and halo nitrotoluene, as described, for example, in U.S. Patents Nos. 4,050,993; 4,086,147; 4,132,602 and 4,132,603.

We have now found that the formation of the proliferous polymer during the catalytic dehydrogenation of p-ethyltoluene may be inhibited by the addition of a minor amount of $H_2S$ to the effluent from the catalytic dehydrogenation of p-ethyltoluene.

According to the present invention we therefore provide a method for dehydrogenating p-ethyltoluene to p-methylstyrene in the presence of steam at 550—700°C by passing a mixture of steam and p-ethyltoluene over a self-regenerative steam dehydrogenation catalyst, in which the formation of proliferous polymer in the cooling train is inhibited by continuously adding $H_2S$ to the dehydrogenation effluent entering the cooling train.

The feed to the dehydrogenation is preferably an ethyltoluene isomer mixture consisting essentially of 90 to 99 weight percent of the para isomer and 1 to 10 weight percent of the meta isomer. The mixture is substantially free of the undesirable ortho isomer and this isomer usually constitutes less than 0.1 weight percent of the mixture. Such mixtures and their preparation are described in U.S. Patents Nos. 4,086,287 and 4,143,084.

The catalysts which can be used in the dehydrogenation are the self-regenerative system dehydrogenation catalysts well known in the art. Some have been based primarily on ferric oxide or copper chromite with potassium added, usually in the form of potassium carbonate, to promote the water-gas reaction. Representative prior art showing such catalysts includes U.S. Patents Nos. 2,870,228; 2,916,531; 3,179,706 and 3,703,593.

Other catalysts of this general type are based upon a mixture of ferric oxide and zinc oxide, as described in U.S. Patents Nos. 3,205,179 and 3,907,916. In another embodiment, the ferric oxide can be in the form of a mixture of yellow (hydrated) and red (dehydrated) ferric oxides, such as described in U.S. Patent No. 3,703,593.

In general, water in the form of steam is admixed with vaporized ethyltoluene in a steam to ethyltoluene weight ratio of from 1.5 to 4. The ethyltoluene is fed at a liquid hourly space velocity (LHSV) of from 0.2 to 3. The dehydrogenation reaction may be carried out semi-continuously or continuously, using a fixed bed of self-regenerative steam dehydrogenation catalyst. The dehydrogenation reaction is carried out at temperatures from 550°C to 700°C.

The dehydrogenation reaction produces a methylstyrene isomer mixture having an isomer distribution corresponding to that of the feed, i.e., 90 to 99 weight percent of the para isomer and 1 to 10 percent of the meta isomer and which is substantially free of the undesirable ortho isomer, usually less than 0.1 weight percent. Because of the predominance of the para isomer, the mixture is conveniently called p-methylstyrene. The effluent also contains unreacted feed, which can be recycled, and water.

The formation of proliferous polymer formation is inhibited by continuously adding a minor amount of $H_2S$ to the dehydrogenation effluent entering the cooling train. Generally, the amount of $H_2S$ added will be from 0.5 cc to 8 cc per minute for 100 cc per hour of p-ethyltoluene.

Comparison example

Into a reaction tube provided with means for feeding a vaporized mixture of steam and hydrocarbon and with a condenser and product recovery means, there was placed 100 cc of a $Fe_2O_3$—$K_2CO_3$—$Cr_2O_3$ catalyst bed (76 wt. % $Fe_2O_3$, 20 wt. % $K_2CO_3$, 4 wt.% $Cr_2O_3$). A mixture of steam and hydrocarbon (97 wt.% p-ethyltoluene, 3 wt.% m-ethyltoluene) was continuously fed at an LHSV of water of 1.6 and an LHSV of hydrocarbon of 1.0. The catalyst bed temperature was 620°C. After several hours of operation, the condenser plugged up, due to formation and build-up of polymer. Before the condenser plugged, conversion was 72% and selectivity was 87.5%.

Example 1

The run described in the Comparison Example was repeated, except that $H_2S$ was introduced continuously at a rate of 1 cc per minute into the dehydrogenation effluent as it entered the condenser. There was no plugging of the condenser by polymer formation.

## Claim

A method for dehydrogenating p-ethyltoluene to p-methylstyrene in the presence of steam at 550—700°C by passing a mixture of steam and p-ethyltoluene over a self-regenerative steam dehydrogenation catalyst characterised in that H$_2$S is continuously added to the dehydrogenation effluent entering the cooling train in an amount to inhibit proliferous polymer formation in the cooling train.

## Patentanspruch

Ein Verfahren zur Dehydrierung von p-Ethyltoluol zu p-Methylstyrol in Gegenwart von Dampf bei 550—700°C, bei dem eine Mischung aus Dampf und p-Ethyltoluol über einen selbstregenerierenden Dampf-Dehydrierungskatalysator geleitet wird, dadurch gekennzeichnet, daß den die Dehydrierung verlassenden und in den Kühlteil eintretenden Produkten kontinuierlich H$_2$S in einer solchen Menge zugesetzt wird, daß eine Bildung eines wuchernden Polymeren in dem Kühlteil unterdrückt wird.

## Revendication

Procédé pour déshydrogéner du p-éthyltoluène en p-méthylstyrène en présence de vapeur d'eau à 550—700°C par passage d'un mélange de vapeur d'eau et de p-éthyltoluène sur un catalyseur de déshydrogénation autogénérateur de vapeur, caractérisé en ce que le H$_2$S est ajouté en continu à l'effluent de déshydrogénation qui pénètre dans le système de refroidissement, à une quantité pour empêcher la formation de polymère prolifère dans le système de refroidissement.